# EUROPÄISCHE PATENTANMELDUNG

(11) **EP 1 757 272 A2**
(43) Veröffentlichungstag der Anmeldung: **28.02.2007**
(21) Anmeldenummer: 06016894.5
(22) Anmeldetag: 12.08.2006
(51) Int. Cl.: A61K 9/16, A61K 9/00, A61K 31/7036, A61P 19/00, A61L 27/16, A61L 27/50

(54) **Wirkstofffreisetzungssystem und seine Verwendung**

(30) Priorität: 25.08.2005 DE 102005040429
(71) Anmelder: Heraeus Kulzer GmbH, 63450 Hanau (DE)
(72) Erfinder: Kühn, Klaus-Dieter, Dr., 35041 Marburg (DE); Vogt, Sebastian, Dr., 99084 Erfurt (DE)
(74) Vertreter: Kühn, Hans-Christian

(57) **Zusammenfassung**

Es wird ein lokales Wirkstofffreisetzungssystem beschrieben, das aus kugelförmigen Körpern besteht, die aus Polymethylmethacrylat oder Polymethylmethacrylat-co-methylacrylat und ggf. Zirkoniumdioxid und/oder Bariumsulfat und einem pharmazeutischen Wirkstoff aufgebaut sind, das mindestens eine bis mindestens 120 °C stabile, hämostyptisch wirksame Verbindung, vorzugsweise ein Calciumsalz, enthält. Das lokale Wirkstofffreisetzungssystem wird als Medizinprodukt oder als Arzneimittel bereitgestellt.

## Beschreibung

Der Gegenstand der Erfindung ist ein lokal wirksames Wirkstofffreisetzungssystem, das aus Körpern besteht, die im Wesentlichen aus Polymethylmethacrylat oder Polymethylmethacrylat-co-methylacrylat, Zirkoniumdioxid oder Bariumsulfat und einem pharma-zeutischen Wirkstoff aufgebaut sind.

Auch gegenwärtig stellt die Behandlung der Osteomyelitis eine der schwierigsten Herausforderungen der Knochenchirurgie dar. Osteomyelitis kann hämatogen, posttraumatisch oder postoperativ entstehen. Besonders schwierig ist die chronische Verlaufsform der Osteomyelitis zu behandeln, die im Extremfall zu Verlust von Gliedmaßen und bis zur Sepsis führen kann.

Üblich ist die chirurgische Sanierung durch radikales Debridment. Dabei wird der infizierte bzw. nekrotische Knochen weiträumig abgetragen. Im Folgenden wird die Knochenkavität mit einem lokalen Antibiotika-Träger ausgefüllt oder durch eine wiederholte Saug-Spül-Drainage behandelt. Durch lokale Freisetzung hoher Antibiotika-Mengen werden bei Verwendung eines hinreichend knochengängigen bakteriziden Antibiotikums, wie Gentamicinsulfat und Clindamycinhydrochlorid, auch die in den anliegenden Knochenarealen verbliebenen bakteriellen Keime wirksam bekämpft.

Kugelförmige lokale Wirkstofffeisetzungssysteme, die aus Polymethylmethacrylat, Zirkoniumdioxid und einem Antibiotikum aufgebaut sind, wurden erstmals 1975 von Klaus Klemm beschrieben (DE 23 20 373). Dieses Konzept erwies sich prinzipiell als erfolgreich, nachteilig war jedoch, dass nur ein geringer Teil des in den Kugeln enthaltenden Wirkstoffes freigesetzt wurde.

In Weiterentwicklung dieser Wirkstoffträger wurde 1978 von Heuser und Dingeldein vorgeschlagen, Glycin oder andere Aminosäuren zur Verbesserung der Antibiotikum-Freisetzung zuzusetzen (DE 26 51 441). Die inkorporierten Aminosäuren gehen nach Kontakt mit Wundsekret in Lösung und bilden Porensysteme, aus denen der Wirkstoff heraus diffundieren kann. Dadurch wurde eine verbesserte Wirkstofffreisetzung erreicht.

Lokale Wirkstofffreisetzungssysteme, die hauptsächlich aus Polymethylmethacrylat, einem Röntgenopaker und einem Antibiotikum aufgebaut sind, lassen sich entweder durch ein spezielles Spritzgießverfahren (DE 23 20 373) oder auch durch Gießen von Antibiotikum enthaltenden Polymethylmethacrylat-Knochenzementen in speziellen Formen herstellen (EP 0 796 712).

Gegenwärtig wird ein lokales Wirkstofffreisetzungssystem, das aus Kugeln besteht, die aus Polymethylmethacrylat, Zirkoniumdioxid, Glycin und Gentamicin aufgebaut sind, die miteinander durch einen polyfilen, chirurgischen Stahldraht verbunden sind, von der Firma Heraeus Kulzer GmbH produziert und von der Firma Biomet unter dem Namen Septopal^{®} vertrieben.

Es wurde vielfach von Anwendern dieses lokalen Wirkstofffreisetzungssystems die Beobachtung berichtet, dass die lokale Antibiotikum-Therapie besonders erfolgreich ist, wenn sich unmittelbar nach der Applikation der Wirkstoffträger ein Hämatom um die Wirkstoffträger ausbildet. Diese Beobachtung ist dadurch zu erklären, dass das geronnene Blut die Diffusion des Gentamicins verzögert und damit den Abtransport des Wirkstoffes behindert. Dadurch verbleibt das Gentamicin über einen längeren Zeitraum in der zuvor chirurgisch sanierten Knochenkavität und bekämpft dadurch lang anhaltend die restlichen bakteriellen Keime.

Der Erfindung liegt die Aufgabe zu Grunde, ein Wirkstofffreisetzungssystem zu entwickeln, das einerseits eine retardierte Wirkstofffreisetzung zeigt und andererseits die Blutgerinnung in der unmittelbaren Umgebung der Wirkstoffträger begünstigt.

Die Aufgabe wurde in der Weise gelöst, dass ein (lokales) Wirkstofffreisetzungssystem entwickelt wurde, das aus Körpern besteht, die im Wesentlichen aus Polymethylmethacrylat oder Polymethylmethacrylat-co-methylacrylat, Zirkoniumdioxid oder Bariumsulfat und einem pharmazeutischen Wirkstoff aufgebaut sind, die jedoch dadurch charakterisiert sind, dass mindestens eine bis 120 °C stabile, hämostyptisch wirksame Verbindung enthalten ist. Durch die hämostyptisch wirksame Verbindung wird die Bildung von Hämatomen begünstigt. Es ist wesentlich für die Erfindung, dass diese Verbindung mindestens bis 120 °C stabil ist, damit eine Herstellung der Wirkstoffträger durch Spritzgießen möglich ist. Die Körper können bevorzugt kugelförmig sein.

Bevorzugt sind als hämostyptische wirksame Verbindungen anorganische oder organische Calciumsalze. Es ist eine an sich bekannte Tatsache, dass gelöste Calcium-lonen die Blutgerinnung beschleunigen können. Calcium-lonen sind ein essentieller Bestandteil an mehreren Stellen der Gerinnungskaskade. Sie wirken mit bei der Aktivierung von Faktor VII und Faktor IX und damit bei der Bildung des Prothrombinaktivators. Calcium-lonen sind weiterhin essentiell bei der Einwirkung von Thrombin auf Fibrinogen unter Bildung von Fibrinmonomeren, die ihrerseits unter Mitwirkung des aktivierten Faktors XIII das Fibrinnetz ausbilden.

Die mindestens eine hämostyptisch wirksamen Verbindung ist vorzugsweise zu 0,1-60,0 Masseprozent, bezogen auf die kugelförmigen Körper enthalten.

Handelt es sich um Calciumsalze, dann sollten diese bei Raumtemperatur eine Löslichkeit in Wasser von mindestens 0,5 g pro Liter besitzen.

Die Calciumsalze Calciumsulfat, Calciumsulfat-Dihydrat, Calciumsulfat-Hemihydrat, Calciumhydroxid, Calciumdihydrogenphosphat, Calciumlactat, Calciumglukonat und Calciumacetat sind besonders bevorzugt. Daneben können auch andere pharmazeutisch akzeptable Calciumsalze Verwendung finden. So ist es ebenfalls möglich, auch Calciumsalze von Aminosäuren, Aldonsäuren und Uronsäuren zu verwenden.

Das jeweilige Calciumsalz kann mikroporös sein. Besonders wird mikroporöses Calciumsulfat-Dihydrat bevorzugt, ganz besonders mikroporöses Calciumsulfat-Dihydrat, in dessen mikroporösem Hohlraumsystem ein pharmazeutischer Wirkstoff aus den Gruppen der Antibiotika, Antiphlogistika, der Hormone und Cancerostatika enthalten ist. Diese Wirkstoffe können zum Beispiel durch Tränken in das Calciumsulfat-Dihydrat eingebracht werden. Es ist auch möglich, direkt in das mikroporöse Calciumsulfat-Dihydrat in Wasser gering lösliche Wirkstoffsalze auszufällen.

Das Calciumsalz kann Zirkoniumdioxid oder Bariumsulfat vollständig ersetzen. Das Wirkstofffreisetzungssystem ist dann nur noch aus dem Polymethylmethacrylat oder Polymethylmethacrylat-co-methylacrylat, dem Calciumsalz und dem Wirkstoff aufgebaut. Das Calciumsalz erfüllt prinzipiell auch die Funktion eines Röntgenopakers. Jedoch ist die Absorption der Röntgenstrahlung deutlich geringer ausgeprägt als bei Zirkoniumdioxid oder Bariumsulfat. Das Wirkstofffreisetzungssystem wird durch das Polymethylmethacrylat oder Polymethylmethacrylat-co-methylacrylat zusammengehalten.

Die Verwendung erfolgt für gewöhnlich in der Weise, dass das lokale Wirkstofffreisetzungssystem als Medizinprodukt oder als Arzneimittel hergestellt oder bereitgestellt wird.

Die Erfindung wird durch nachstehende Beispiele erläutert, ohne jedoch die Erfindung zu beschränken.

### Beispiel 1

Es wird ein Gemisch aus 854,0 g Polymethylmethacrylat-co-methylacrylat (Molmasse ~900.000 g/mol), 89,0 g Zirkoniumdioxid, 42,0 g Gentamicinsulfat (Aktivitätskoeffizient 600), 10,0 g Glycin und 5,0 g Calciumsulfat-Dihydrat durch intensives Vermahlen hergestellt. Aus diesem Gemisch werden mit Hilfe einer Spritzgießvorrichtung auf einen polyfilen, chirurgischen Stahldraht annähernd kugelförmige Körper mit einem Durchmesser von 7 mm gespritzt. Diese Körper haben eine Masse von 240 mg.

### Beispiel 2

Es wird ein Gemisch aus 854,0 g Polymethylmethacrylat-co-methylacrylat (Molmasse ∼900.000 g/mol), 89,0 g Zirkoniumdioxid, 42,0 g Gentamicinsulfat (Aktivitätskoeffizient 600), 5,0 g Glycin und 10,0 g Calciumsulfat-Dihydrat durch intensives Vermahlen hergestellt. Aus diesem Gemisch werden mit Hilfe einer Spritzgießvorrichtung auf einen polyfilen, chirurgischen Stahldraht annähernd kugelförmige Körper mit einem Durchmesser von 7 mm gespritzt. Diese Körper haben eine Masse von 240 mg.

### Beispiel 3

Es wird ein Gemisch aus 769,0,0 g Polymethylmethacrylat-co-methylacrylat (Molmasse ~900.000 g/mol), 89,0 g Zirkoniumdioxid, 42,0 g Gentamicinsulfat (Aktivitätskoeffizient 600), und 100,0 g Calciumsulfat-Dihydrat durch intensives Vermahlen hergestellt. Aus diesem Gemisch werden mit Hilfe einer Spritzgießvorrichtung auf einen polyfilen, chirurgischen Stahldraht annähernd kugelförmige Körper mit einem Durchmesser von 7 mm gespritzt. Diese Körper haben eine Masse von 240 mg.

### Beispiel 4

Es wird ein Gemisch aus 854,0 g Polymethylmethacrylat-co-methylacrylat (Molmasse ∼900.000 g/mol), 42,0 g Gentamicinsulfat (Aktivitätskoeffizient 600) und 104,0 g Calciumsulfat-Dihydrat durch intensives Vermahlen hergestellt. Aus diesem Gemisch werden mit Hilfe einer Spritzgießvorrichtung auf einen polyfilen, chirurgischen Stahldraht annähernd kugelförmige Körper mit einem Durchmesser von 7 mm gespritzt. Diese Körper haben eine Masse von 240 mg.

## Patentansprüche

1. Wirkstofffreisetzungssystem, das aus Körpern besteht, die im Wesentlichen aus Polymethylmethacrylat oder Polymethylmethacrylat-co-methylacrylat sowie ggf. Zirkoniumdioxid und/oder Bariumsulfat und ferner einem pharmazeutischen Wirkstoff aufgebaut sind, **dadurch gekennzeichnet, dass** mindestens eine bis mindestens 120 °C stabile, hämostyptisch wirksame Verbindung enthalten ist.

2. Wirkstofffreisetzungssystem nach Anspruch 1, **dadurch gekennzeichnet, dass** 0,1-60,0 Masseprozent mindestens einer bis mindestens 120 °C stabilen, hämostyptisch wirksamen Verbindung enthalten ist.

3. Wirkstofffreisetzungssystem nach den Ansprüchen 1 und 2, **dadurch gekennzeichnet, dass** als hämostyptisch wirksame Verbindung mindestens ein anorganisches oder organisches Calciumsalz enthalten ist.

4. Wirkstofffreisetzungssystem nach den Ansprüchen 1 und 2, **dadurch gekennzeichnet, dass** das jeweilige Calciumsalz bei Raumtemperatur eine Löslichkeit in Wasser von mindestens 0,5 g pro Liter besitzt.

5. Wirkstofffreisetzungssystem nach einem der Ansprüche 1 bis 4, **dadurch gekennzeichnet, dass** das jeweilige Calciumsalz der Gruppe Calciumsulfat, Calciumsulfat-Dihydrat, Calciumsulfat-Hemihydrat, Calciumdihydrogenphosphat, Calciumlactat, Calciumglukonat und Calciumacetat angehört.

6. Wirkstofffreisetzungssystem nach einem der Ansprüche 1 bis 5, **dadurch gekennzeichnet, dass** das jeweilige Calciumsalz mikroporös ist.

7. Wirkstofffreisetzungssystem nach einem der Ansprüche 1 bis 6, **dadurch gekennzeichnet, dass** das Calciumsalz mikroporöses Calciumsulfat-Dihydrat ist, in dessen mikroporösem Hohlraumsystem ein pharmazeutischer Wirkstoff aus den Gruppen der Antibiotika, Antiphlogistika, der Hormone und Cancerostatika enthalten ist.

8. Wirkstofffreisetzungssystem nach einem der Ansprüche 1 bis 7, **dadurch gekennzeichnet, dass** das Calciumsalz das Zirkoniumdioxid oder Bariumsulfat vollständig ersetzt.

9. Verwendung eines Wirkstofffreisetzungssystems nach einem der Ansprüche 1 bis 8 zur Herstellung oder Bereitstellung eines Medizinprodukts oder Arzneimittels.
